# FASCICULE DE BREVET EUROPEEN

(11) **EP 4 152 922 B1**
(45) Date de publication et mention de la délivrance du brevet: **08.05.2024**
(21) Numéro de dépôt: 21727438.0
(22) Date de dépôt: 18.05.2021
(51) Int. Cl.: A01K 67/033

(54) **DISPOSITIF DE TRI D'INSECTES PERMETTANT LA SÉPARATION D'INSECTES RAMPANTS DU RESTE D'UN MÉLANGE**
VORRICHTUNG ZUM SORTIEREN VON INSEKTEN, DIE DAS ABTRENNEN VON KRIECHENDEN INSEKTEN AUS DEM REST EINER MISCHUNG ERMÖGLICHT
DEVICE FOR SORTING INSECTS, ALLOWING THE SEPARATION OF CRAWLING INSECTS FROM THE REST OF A MIXTURE

(30) Priorité: 19.05.2020 FR 2005102
(43) Date de publication de la demande: 29.03.2023
(73) Titulaire: Ynsect, 91058 Évry-Courcouronnes Cedex (FR)
(72) Inventeur: ESCALANTE, Pedro, 91260 JUVISY SUR ORGE (FR); MICHEL, Cyril, 91510 JANVILLE SUR JUINE (FR); CHATEAU, Mathieu, 91000 EVRY (FR); CHOGNE, Manon, 21000 DIJON (FR); BERRO, Fabrice, 75001 PARIS (FR); ESCAROZ CETINA, Arturo, 5611BN EINDHOVEN (NL); SARTON DU JONCHAY, Thibault, 60710 CHEVRIÈRES (FR)
(74) Mandataire: Santarelli
(86) Numéro de dépôt international: PCT/EP2021/063199
(87) Numéro de publication internationale: WO 2021/233945

(56) Documents cités:
- WO-A1-2019/084554
- CN-A- 105 501 833
- CN-A- 109 850 487
- JP-A- H1 159 853
- KR-A- 20200 040 440
- KR-B1- 101 464 734

## Description

La présente invention concerne le domaine du tri des insectes, notamment dans le cadre d'un élevage d'insectes à échelle industrielle.

Les insectes visés par l'invention sont les insectes rampants ou essentiellement rampants, par exemple les coléoptères, les isoptères, blattoptères, les hémiptères, les hétéroptères.

Elle trouve une application préférentielle dans le tri des ténébrions meuniers, également appelé tenebrio molitor.

Sauf précision contraire, le terme « insecte » est employé pour désigner tout stade d'évolution de l'oeuf ou oothèque à l'insecte adulte. Comme cela est détaillé ci-après, l'invention porte sur le tri d'un mélange contenant des insectes adultes ou des larves dotées de pattes afin de les séparer du reste de ce mélange.

La production d'insectes à grande échelle présente de nombreux intérêts, notamment dans l'agro-industrie. Certaines espèces d'insectes comestibles sont en effet riches en protéines et peuvent servir notamment à l'alimentation animale, pour les poissons, les crustacés, et certains volatiles. L'élevage d'insectes à grande échelle présente aussi des intérêts dans d'autres domaines industriels. Par exemple, l'exosquelette des insectes est constitué en grande partie de chitine, dont un dérivé connu est le chitosan. Les applications de la chitine et/ou du chitosan sont nombreuses : cosmétique (composition cosmétique), médicale et pharmaceutique (composition pharmaceutique, traitement des brûlures, biomatériaux, pansements cornéens, fils chirurgicaux), diététique et alimentaire, technique (agent filtrant, texturant, floculant ou adsorbant notamment pour la filtration et dépollution de l'eau), etc.

Le document FR3034622 présente un atelier adapté à l'élevage d'insectes à grande échelle, c'est-à-dire à échelle industrielle. L'élevage y met en oeuvre des contenants d'élevage (typiquement des bacs) qui sont empilés pour former des unités élémentaires d'élevage. Les unités élémentaires d'élevage sont stockées dans une première zone, et, lorsqu'une opération d'élevage doit être réalisée, les contenants sont amenés vers un poste adapté à la réalisation de l'opération, groupés en unités élémentaires d'élevage ou dégroupés unitairement.

Les opérations d'élevage concernent de manière non exhaustive le nourrissage, l'apport d'eau, la calibration des insectes, l'ajout d'insectes dans des contenants d'élevage, et de nombreuses et diverses opérations de tri permettant de séparer ou de classer, lors de l'élevage, les insectes selon leur stade d'évolution, ou de séparer les insectes vivants des insectes morts et/ou de leur milieu d'élevage, etc.

Dans ce contexte, il se révèle en particulier complexe de trier les insectes vivants des insectes morts, ou les insectes adultes des insectes à des stades immatures. Les méthodes de tri généralement envisagées sont des méthodes manuelles qui ne permettent pas un tri efficace et rapide à grande échelle.

Des solutions imparfaites à cette problématique sont proposées dans le document WO2019084554. Ce document divulgue des équipements pour le tri d'insectes, fondés sur le principe selon lequel les insectes rampants ont la faculté de s'agripper par leurs pattes à une surface sur laquelle ils évoluent. Les équipements proposés dans ce document sont néanmoins complexes à mettre à oeuvre industriellement et/ou se révèlent imparfaits dans leur efficacité de tri.

Les documents KR101464734 et EP3415002 divulguent également des équipements de tri fondés sur ce principe, qui se révèlent également peu efficaces.

L'invention développée vise à proposer un dispositif de tri d'insectes, en particulier pour séparer les insectes adultes ou les larves d'une part d'insectes à d'autres stades de croissance et/ou des éléments inertes (insectes morts, déjections, milieu d'élevage) d'autre part, qui soit applicable industriellement et efficace.

Ainsi, l'invention porte sur un dispositif de tri d'insectes, comportant un convoyeur à bande présentant une surface supérieure et une surface inférieure formée par la bande du convoyeur. Le dispositif comporte un dispositif d'entraînement configuré pour entraîner la bande du convoyeur, de sorte que lors du fonctionnement du convoyeur la bande défile sur la surface supérieure d'une première extrémité vers une deuxième extrémité du convoyeur où la bande est retournée pour passer sur la surface inférieure du convoyeur sur laquelle elle défile de la deuxième extrémité vers la première extrémité où la bande est retournée pour passer de nouveau sur la surface supérieure. Le dispositif comporte aussi un dispositif permettant le dépôt d'un mélange comportant des insectes sur la surface supérieure du convoyeur à bande, de sorte que lors du retournement de la bande au niveau de la deuxième extrémité tout ou partie des insectes restent accrochés à la bande tandis que le reste du mélange tombe par gravité. Le dispositif comporte un dispositif de séparation des insectes permettant de détacher les insectes accrochés sur la surface inférieure du convoyeur.

Dans le dispositif de tri d'insectes, la surface supérieure du convoyeur étant sensiblement plate et horizontale sur une majorité de sa longueur entre la première extrémité et la deuxième extrémité, ladite surface supérieure du convoyeur comporte une portion extrême, adjacente à la deuxième extrémité, présentant une inclinaison vers le bas, comprise entre 20° et 90° vis-à-vis du reste de la surface supérieure du convoyeur.

L'invention propose ainsi un système de tri efficace pour séparer les insectes dotés de pattes du reste d'un mélange. Ce dispositif est fondé sur la faculté des insectes dotés de pattes à s'accrocher à la surface sur laquelle ils évoluent. Cette surface peut être formée par la surface extérieure d'un convoyeur à bande ayant des propriétés appropriées. Lors du retournement de la bande du convoyeur, c'est-à-dire lors de son passage de la surface supérieure du convoyeur vers la surface inférieure du convoyeur, les insectes restent agrippés sur la surface de la bande, c'est-à-dire sur sa face extérieure, tandis que le reste du mélange qui est trié tombe par gravité. Ce principe de tri par convoyeur à bande sur lequel s'agrippent les insectes, qui est connu dans l'état de la technique, est fortement amélioré par la présence d'une portion extrême présentant une inclinaison. Cette inclinaison a plusieurs effets bénéfiques sur la qualité du tri, c'est-à-dire sur la proportion d'insectes adultes restant agrippés à la bande du convoyeur lors de son retournement. Principalement, en provoquant un début de chute ou de glissement des insectes sur la bande, elle les incite à s'y agripper, de sorte que lors du retournement de la bande, les insectes n'ont pas à s'agripper très rapidement à celle-ci. De manière générale, cette pente ou inclinaison limite le caractère soudain du retournement de la bande, ce qui offre par exemple plus de temps aux insectes pour s'agripper. Même lorsqu'une pente à 90° ou proche de 90° est employée, cela limite l'accélération subie par les insectes du fait du changement de direction de la bande, et permet une meilleure accroche des insectes. En outre, la pente permet à certains insectes qui auraient été transportés sur convoyeur sur leur dos, sans avoir la possibilité de s'agripper à la bande, de se retourner sur leurs pattes.

Ce faisant, le tri est amélioré et/ou la vitesse de défilement de la bande peut être accélérée.

L'inclinaison peut être plus particulièrement comprise entre 30° et 70°, de préférence entre 45° et 60°. La portion extrême du convoyeur présentant une inclinaison peut avoir par exemple une longueur comprise entre vingt centimètres et un mètre, mesurée le long de sa surface supérieure Le dispositif peut comporter un moyen de réglage de l'inclinaison.

La pente peut ainsi être adaptée au tri réalisé, et être optimisée selon de multiples paramètres : l'espèce des insectes triés, leur stade d'évolution, le contenu du mélange à trier, le type de bande employée sur le convoyeur, la vitesse de défilement de la bande, etc.

La bande peut comporter une face extérieure présentant une structure adaptée à l'accroche des pattes des insectes.

La bande peut comporter deux couches distinctes, une couche formant la face extérieure pour l'accroche des pattes des insectes et une couche intérieure de renfort de la bande.

La couche extérieure de la bande peut être formée d'un matériau tissé. Ce matériau tissé peut présenter des mailles régulières sensiblement carrées ayant une ouverture de 110 microns à 1000 microns de côté.

La bande employée sur le convoyeur à bande est importante pour la qualité du tri. La face extérieure de la bande (c'est-à-dire la face visible de la bande, qu'elle soit au-dessus ou au-dessous du convoyeur) sert en effet de surface d'accroche pour les insectes. Elle doit permettre cette accroche, grâce à une rugosité et/ou une texture de surface adaptée. L'emploi d'un tissu, par exemple un tissu de polyester ou de polyamide (PA), donne de bons résultats. L'épaisseur des fils et leur nombre, définissant la taille des mailles, est important. Il convient en effet que les mailles soient adaptées à la taille des crochets présents aux extrémités des pattes des insectes devant être triés. Le crochet d'une patte de ténébrion meunier adulte mesure par exemple de l'ordre de 0,3mm. Les larves ont des crochets plus petits.

Le dispositif de séparation peut être une lame ou une brosse s'étendant transversalement vis-à-vis de la bande, à proximité immédiate de sa surface.

Un moyen mécanique de séparation des insectes qui sont agrippés à la bande permet une séparation efficace, sans blesser les insectes ni abîmer la bande du convoyeur.

Le dispositif peut comporter un système de nettoyage de la bande générant une lame d'air comprimé venant impacter la bande du convoyeur au niveau de sa surface inférieure, entre le dispositif de séparation et la première extrémité du convoyeur.

Le nettoyage de la bande est important pour maintenir l'état de surface de la bande, qui est fondamental pour la qualité du tri. En effet, la face extérieure de la bande servant de surface d'accroche pour les insectes, il est important que sa rugosité, la texture de sa surface, ne soit pas altérée dans le temps. Cela passe par un nettoyage, un dé-colmatage, régulier de la bande. Mais il faut également que ce nettoyage n'altère pas les propriétés de la bande par usure mécanique, notamment par abrasion. La sélection parmi les technologies de nettoyage par air comprimé permet de répondre à cette problématique.

Le dispositif peut comporter en outre une hotte aspirante adaptée à aspirer de l'air présent au-dessus de la surface supérieure du convoyeur. La présence d'une hotte aspirante permet la récupération des poussières et des particules les plus légères présentes dans le mélange à trier. Dans une certaine mesure, elle permet de nettoyer les insectes des fines particules qu'ils sont susceptibles de porter.

Le dispositif peut comporter un carter enveloppant le convoyeur de sorte à limiter la propagation de poussières depuis le convoyeur vers l'extérieur du carter. Cela est particulièrement avantageux lorsqu'un nettoyage de la bande par air comprimé est employé.

L'invention porte également sur une machine de tri d'insectes comportant un premier dispositif de tri tel que précédemment décrit et un deuxième dispositif tel que précédemment décrit, dans laquelle ledit reste du mélange tombant par gravité du convoyeur du premier dispositif de tri tombe directement sur, ou est amené sur, la surface supérieure du convoyeur du deuxième dispositif.

La succession de plusieurs convoyeurs à bande pour réaliser le tri permet d'augmenter la proportion d'insectes récupérés dans le mélange trié. Avec le dispositif proposé dans l'invention, correctement optimisé, la demanderesse a obtenu un taux de récupération des insectes dans le mélange de l'ordre de 85% en masse avec un convoyeur à bande unique, et de l'ordre de 95% en masse avec deux convoyeurs successifs. Ce taux est satisfaisant pour un élevage à grande échelle. Bien évidemment, plus de deux convoyeurs successifs peuvent être employés pour améliorer encore plus le taux de récupération des insectes.

L'invention porte aussi sur l'utilisation d'un dispositif tel que précédemment décrit ou d'une machine telle que précédemment décrite pour la récupération des insectes adultes dans un mélange comportant des insectes adultes et des larves d'insectes.

L'invention porte aussi sur un dispositif tel que précédemment décrit ou d'une machine telle que précédemment décrite pour la récupération des insectes vivants dans un mélange comportant des insectes vivants et des insectes morts.

Les insectes triés peuvent par exemple être des ténébrions meuniers.

D'autres particularités et avantages de l'invention apparaîtront encore dans la description ci-après.

Aux dessins annexés, donnés à titre d'exemples non limitatifs :
la figure 1 représente, selon une vue schématique en trois dimensions, un dispositif de tri conforme à un premier exemple de mode de réalisation de l'invention ;
la figure 2 représente, selon un schéma de principe, les flux dans une machine de tri conforme à un exemple de mode de réalisation de l'invention ;
la figure 3 représente, selon une vue schématique en trois dimensions, les éléments principaux d'une machine conforme à un mode de réalisation de l'invention ;
la figure 4 représente, selon un plan simplifié en vue de dessus, une machine correspondant à la machine de la figure 3 ;
la figure 5 représente, selon un plan simplifié en vue de côté, la machine des figures 3 et 4.

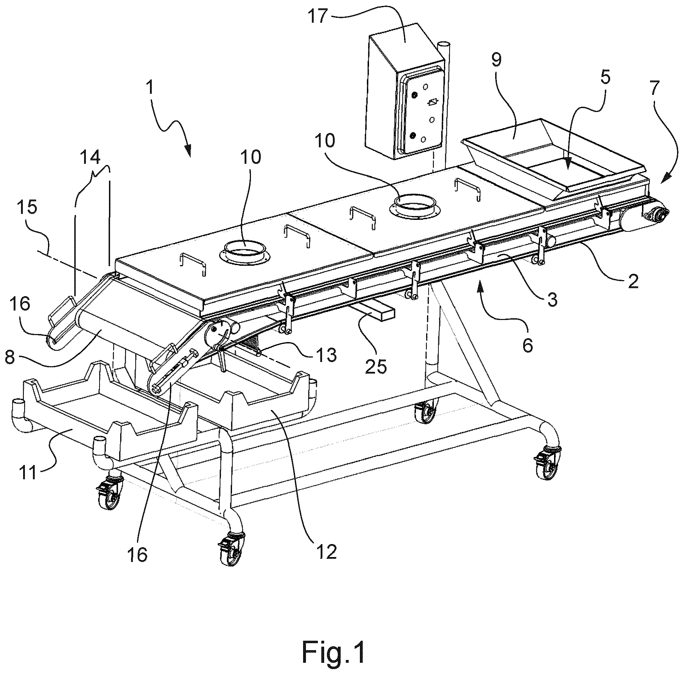

La figure 1 représente un dispositif de tri 1 conforme à un premier exemple de mode de réalisation de l'invention. Le dispositif de tri comporte un convoyeur 2 à bande, qui comporte une bande 3 entraînée par un dispositif d'entraînement 4 permettant la mise en mouvement de la bande 3. Le dispositif d'entraînement 4 comporte typiquement un moteur qui met en rotation un arbre ou un rouleau d'entraînement de la bande 3.

La bande 3 forme une boucle fermée. Elle constitue pour le convoyeur 2 une surface supérieure 5 et une surface inférieure 6. La surface supérieure 5 est la surface située au-dessus du convoyeur permettant le transport d'un produit sur le convoyeur 2, entre une première extrémité 7 du convoyeur et une deuxième extrémité 8 du convoyeur. La surface inférieure 6 correspond à la zone de retour de bande 3 entre la deuxième extrémité 8 du convoyeur 2 et sa première extrémité. Chaque extrémité du convoyeur correspond à une zone de retournement de la bande 3 Au niveau de la première extrémité 7 la bande 3 passe de la surface inférieure 6 à la surface supérieure 5 du convoyeur. Au niveau de la deuxième extrémité 8 la bande 3 passe de la surface supérieure 5 du convoyeur à la surface inférieure 6.

Le dispositif représenté à la figure 1 comporte un moyen facilitant le dépôt d'un produit, notamment d'un mélange contenant des insectes adultes ou des larves dotées de pattes, sur la surface supérieure 5 du convoyeur 2. En l'occurrence, une trémie ouverte, ou plus généralement un bac de dépose 9 facilitant le dépôt d'un produit sur la bande 3 du convoyeur 2 est disposé à proximité de la première extrémité 7 du convoyeur 2. Un dispositif d'alimentation, automatique ou manuel, peut également être prévu.

Le produit déposé sur la surface supérieure 5 du convoyeur à bande, à savoir un mélange comportant des insectes rampants (adultes ou larves dotées de pattes), va ainsi être transporté sur la surface supérieure du convoyeur 2, du fait du défilement de la bande 3, jusqu'à la deuxième extrémité 8. Lors de ce transport, et selon la longueur du convoyeur, le mélange se répartit à la surface du convoyeur et les insectes présents dans le mélange tendent à se remettre sur leurs pattes, s'ils ne l'étaient pas suite au dépôt du mélange sur la bande 3. Ces phénomènes peuvent être promus par des moyens vibrateurs optionnels.

Le temps de transport sur le convoyeur 2 peut également être employé pour dépoussiérer l'air et éliminer une partie des particules les plus fines présentes dans le mélange, à l'aide de hottes aspirantes. Les hottes aspirantes ne sont pas représentées en totalité à la figure 1, mais le mode de réalisation de la figure 1 présente deux embouchures de hottes 10 où l'aspiration est réalisée.

Cette aspiration permet également un certain dépoussiérage des insectes présents dans le mélange.

Lorsque le mélange déposé sur la bande 3 atteint la deuxième extrémité 8 du convoyeur 2, les insectes accrochés à la bande 3 passent sur la surface inférieure 6 du convoyeur 2, tandis que le reste du mélange tombe par gravité. Le reste du mélange tombant par gravité peut être reçu dans un premier bac de récupération 11.

Les insectes accrochés à la bande 3 sont eux récupérés dans un deuxième bac de récupération 12. Pour cela, un dispositif de séparation 13 des insectes est prévu à l'aplomb du deuxième bac de récupération 12. Le dispositif de séparation 13 peut prendre diverses formes. Il s'agit un moyen permettant de détacher les insectes de la bande 3 afin qu'ils en tombent, sans toutefois blesser les insectes et sans que les insectes ne s'accrochent au moyen de séparation. Les meilleurs résultats ont été obtenus avec une lame, c'est-à-dire un élément rigide et fin, disposé sensiblement transversalement par rapport à la bande 3 et à son sens de défilement, et s'étendant à proximité immédiate de la bande 3, préférentiellement sans toutefois la toucher. D'autres dispositifs de séparation peuvent également être employés, telle qu'une brosse, ou un jet d'air comprimé. L'absence de contact avec la bande 3 en évite l'usure mécanique. Il convient néanmoins que le jeu entre la bande et le dispositif de séparation ne permette pas le passage ou le coincement des insectes. Ce jeu doit donc être petit, par exemple de l'ordre du millimètre. Une séparation des insectes de la bande 3 par air comprimé est également envisageable.

Lorsque le dispositif est en condition d'utilisation, la surface supérieure 5 du convoyeur est sensiblement horizontale, sur une majorité de sa longueur, c'est-à-dire sur une majorité de la distance séparant la première extrémité 7 du convoyeur de sa deuxième extrémité. L'orientation sensiblement horizontale de la majorité de la surface supérieure 5 du convoyeur peut être obtenue en disposant le support du dipositif sur un sol sensiblement horizontal. Selon l'invention, une portion extrême du convoyeur est cependant inclinée. En particulier, la portion extrême du convoyeur située au niveau de la deuxième extrémité 8 du convoyeur 2 présente une inclinaison vers le bas, c'est-à-dire que la deuxième extrémité 8 du convoyeur est située plus bas que la majorité de la surface supérieure 5, ou encore que le mélange arrivant sur cette portion du convoyeur va descendre avant d'atteindre la deuxième extrémité 8.

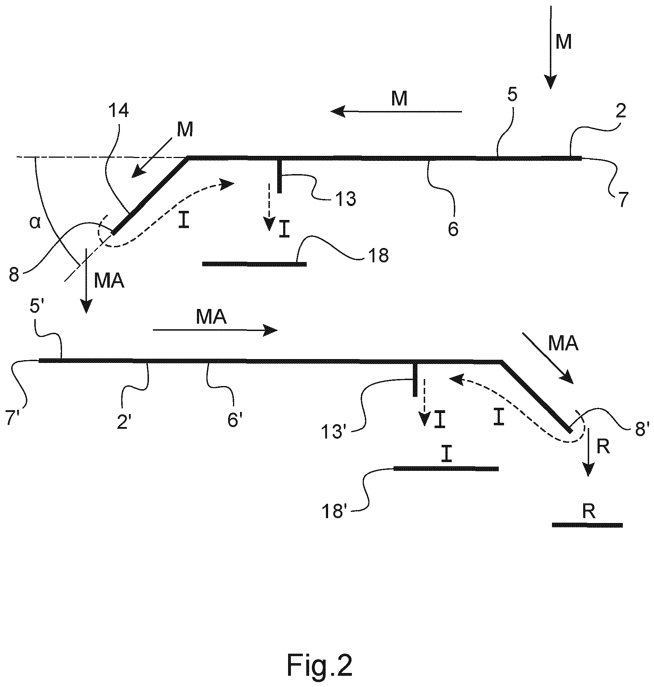

L'inclinaison α de la portion extrême 14 (illustrée à la figure 2) améliore significativement l'efficacité du tri.

Cette inclinaison incite les insectes, par réflexe, à s'agripper à la bande pour éviter leur chute. Elle permet de créer un retournement moins soudain de la bande qu'en l'absence de cette pente, ce qui laisse plus de temps aux insectes pour s'accrocher à la bande, et ce qui réduit l'accélération subie par les insectes lors du retournement de la bande, à tout le moins au début de ce retournement. L'inclinaison permet enfin d'aider certains insectes qui auraient été transportés sur convoyeur sur leur dos, sans avoir la possibilité de s'agripper à la bande, de se retourner sur leurs pattes, du fait de la discontinuité dans le mouvement du mélange à trier créée par la pente.

Dans l'exemple représenté à la figure 1, l'inclinaison α est réglable. Pour cela un axe de basculement 15 permet le basculement, sur une certaine plage d'angles, de la portion extrême 14. Afin de permettre l'ajustement de la pente sans augmenter ou diminuer la tension mécanique dans la bande 3, mais aussi pour le réglage de cette tension, le rouleau extrême du convoyeur 2 situé à la deuxième extrémité 8 est mobile en translation entre deux bras de réglage 16 qui basculent autour de l'axe de basculement 15. Ce dispositif permet également le relâchement de la tension dans la bande 3 afin d'en permettre un remplacement rapide.

L'inclinaison α, qu'elle soit réglable ou non, peut être comprise entre 20° et 90°, par exemple entre 30° et 70°, les meilleurs résultats de tri ayant été obtenus pour une inclinaison comprise entre 45° et 60° par rapport à l'horizontale, c'est-à-dire pour l'exemple représenté par rapport à la majorité de la surface supérieure 5 du convoyeur 2. En tout état de cause, lors de l'utilisation du dispositif, la surface supérieure 5 du convoyeur peut définir conventionnellement l'horizontale.

La longueur de la portion extrême 14, mesurée le long de la surface supérieure du convoyeur, peut être par exemple comprise entre 20 cm et 1 m, par exemple de l'ordre de 30 cm, ce qui permet l'obtention des avantages précités.

Enfin, le dispositif de la figure 1 est ménagé sur une structure de support rigide. La structure de support de l'exemple représenté est une structure simple, mobile, le dispositif étant lui-même relativement simple et léger. Tout autre type de structure de support adaptée peut être employé.

Dans l'exemple représenté, la structure de support permet également de porter les moyens de contrôle et de réglage 17 du dispositif.

Dans un tel dispositif, les paramètres de fonctionnement sont importants, pour assurer une vitesse de tri suffisante pour un élevage à très grande échelle, conjointement à une efficacité souhaitée du tri.

La vitesse de défilement est ainsi adaptée à la fonction de tri des insectes. La vitesse de défilement est configurée pour offrir aux insectes déposés sur le convoyeur à bande un temps suffisant pour s'accrocher sur la surface extérieure de la bande du convoyeur.

Notamment, une vitesse de défilement de la bande de l'ordre 10 mètres par minute à 30 mètres par minute, par exemple de l'ordre de 15 mètres par minute ou de 20 mètres par minute, donne de bons résultats.

La constitution de la bande est également importante. En effet, la bande doit présenter, sur sa face extérieure, un état de surface adapté à l'accroche des insectes. Un état de surface approprié peut être obtenu avec divers tissus industriels, par exemple un tissu de polyester (PES) ou de polyamide. Néanmoins, la bande 3 doit également être résistante à l'usure afin de ne pas devoir procéder à des remplacements trop fréquents. C'est pourquoi un assemblage de deux couches pour constituer la bande peut être avantageux, à savoir une couche pour former la face externe qui est adaptée à l'accroche des insectes, et une couche pour former la face interne de la bande 3 qui est résistante à l'usure, notamment à l'abrasion.

Par exemple, la bande 3 peut comporter une bande en tissu de PES 120/34 (c'est-à-dire présentant des ouvertures de mailles de 120 microns et une surface ouverte de 34% de la surface totale) pour la face extérieure de la bande 3 et une bande en tissu de PES 1000/35 (mailles de 1mm et surface ouverte de 35%) pour la face intérieure de la bande 3.

Le matériau, par exemple le tissu, constituant la face extérieure de la bande 3 peut être adapté selon le tri réalisé avec le dispositif, notamment selon l'espèce d'insectes triés et/ou le stade de croissance des insectes triés. Le matériau et notamment son état de surface doit être adapté à l'accroche des insectes par les crochets de leurs pattes. Pour le ténébrion meunier adulte, qui est l'espèce préférentiellement triée à l'aide de l'invention, ces crochets mesurent environ 0,3mm. Les larves de ténébrions meuniers ont des crochets sensiblement plus petits.

D'autres tissus ont été testés avec succès. Pour le tri de ténébrions meuniers adultes, des tissus formés de fils de 250 microns à 500 microns de diamètres ont été testés avec succès. Des tissus ayant des mailles présentant une ouverture de 650 microns à 1mm, avec une surface ouverte de l'ordre de 50% à 65% de leur surface totale se sont révélés particulièrement appropriés. D'autres tissus sont bien évidemment envisageables.

Pour le tri d'un mélange contenant des larves de ténébrions meuniers, des tissus formés de fils plus fins, de l'ordre de 35 microns à 80 microns, se sont révélés appropriés. Des tissus ayant des mailles présentant une ouverture de 85 microns à 150 microns, en particulier de 110 à 150 microns, avec une surface ouverte de l'ordre de 30% à 50% de leur surface totale se sont révélés particulièrement appropriés. D'autres tissus sont bien évidemment envisageables.

De manière plus générale, pour le tri de larves ou d'insectes adultes, les tissus ayant les propriétés suivantes se sont révélés adaptés : une ouverture des mailles de 110 à 1000 µm, un diamètre des fils constitutifs de 80 à 500 µm, une surface d'ouverture de 31% à 64% de la surface totale.

Des bandes métalliques, formant un maillage analogue, ont également été testées avec succès. Enfin, l'état de surface adapté à l'accroche des insectes peut résulter de la présence d'aspérités, de dents, de poils, ou autres formes de rugosités, à la surface de la bande 3.

En optimisant le dispositif décrit ci-dessus, un taux de séparation d'environ 85% en masse des insectes est obtenu (efficacité obtenue pour la séparation des insectes vivants vis-à-vis des insectes morts, dans un mélange initial comportant de l'ordre de 95% en masse d'insectes vivants et d'insectes morts, et moins de 5% de déjections et de substrat d'élevage et autres éléments similaires). Cela signifie que 85% de la masse d'insectes devant être séparés du mélange (adultes, nymphes dotées de pattes) initialement présente dans le mélange et qui est déposé sur le convoyeur sont correctement séparés de ce mélange par le dispositif.

En jouant sur certains paramètres, par exemple sur la vitesse de défilement de la bande, un taux de séparation légèrement supérieur peut être obtenu, mais ce taux (de l'ordre de 90%) n'est pas totalement satisfaisant dans le cadre d'un élevage à grande échelle performant.

C'est pourquoi il est proposé, selon un deuxième aspect développé dans l'invention, de fournir une machine de tri comportant plusieurs dispositifs fonctionnant selon le principe décrit en référence à la figure 1.

La figure 2 est une figure de principe visant à illustrer les flux dans une telle machine.

Un mélange M comportant des insectes rampants est déposé sur un convoyeur 2 à bande, sur sa surface supérieure 5, à proximité de sa première extrémité 7.

Outre des insectes que l'on souhaite séparer du reste du mélange, le mélange M peut comporter, de manière non exhaustive, des insectes qui sont à d'autres stades de leur évolution que les insectes triés (oeufs, larves, nymphes, lorsque le tri vise à séparer des adultes, oeufs et nymphe lorsque le tri vise à séparer les larves), des insectes morts, des déjections d'insectes, et du substrat d'élevage (c'est-à-dire le produit dans lequel les insectes sont élevés, pouvant contenir la nourriture nécessaire à leur croissance).

Le mélange M est transporté vers la deuxième extrémité 8 du convoyeur. Avant d'atteindre cette deuxième extrémité 8, le mélange atteint la portion extrême 14 inclinée du convoyeur 2, qui aide les insectes à s'agripper à la bande du convoyeur 2.

Au niveau de la deuxième extrémité 8 où la bande du convoyeur se retourne pour passer sur la surface inférieure 6, une part importante des insectes l reste accrochée à la bande du convoyeur, tandis que le reste du mélange, à savoir un mélange appauvri MA en insectes, tombe par gravité.

Les insectes l sont détachés de la bande par un dispositif de séparation 13 et tombent sur une surface de réception 18 (pouvant par exemple correspondre au deuxième bac de récupération 12 de la figure 1). Comme précédemment indiqué, 85% environ des insectes initialement présents dans le mélange M peuvent ainsi être récupérés.

Le mélange appauvri MA tombe quant à lui sur la surface supérieure 5' d'un deuxième convoyeur 2', sur sa bande 3', à proximité de sa première extrémité 7'.

Le mélange appauvri MA est transporté vers la deuxième extrémité 8' du deuxième convoyeur 2'. Avant d'atteindre cette deuxième extrémité 8', le mélange atteint la portion extrême 14' inclinée du deuxième convoyeur 2', qui aide les insectes à s'agripper à la bande du deuxième convoyeur 2'.

Au niveau de la deuxième extrémité 8' où la bande du deuxième convoyeur 2' se retourne pour passer sur la surface inférieure 6', une part importante des insectes l présents dans le mélange appauvri reste accrochée à la bande du deuxième convoyeur 2', tandis que le reste du mélange, à savoir le reste R du mélange appauvri MA, tombe par gravité.

Les insectes l sont détachés de la bande par un deuxième dispositif de séparation 13' et tombent sur une deuxième surface de réception 18' (par exemple un deuxième bac de récupération).

Ce faisant, la proportion globale d'insectes séparés du mélange M initial atteint 95% en masse voire plus.

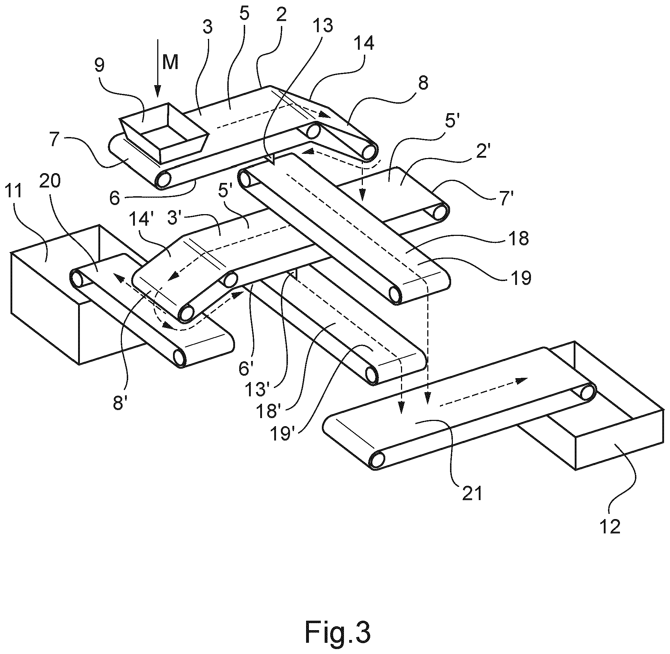

La figure 3 représente, selon une vue schématique en trois dimensions, les éléments principaux d'une machine de tri mettant en oeuvre les flux présentés à la figure 2.

La machine de la figure 3 comporte ainsi un convoyeur 2, doté d'un bac de dépose 9 permettant de déposer le mélange à trier sur la surface supérieure du convoyeur 2, sur sa bande 3. Le tri, à savoir la séparation des insectes présents dans le mélange, est réalisé comme cela est décrit en référence aux figures 1 et 2. De manière
remarquable, la surface de réception 18 pour les insectes séparés par le dispositif de tri est elle-même formée d'un premier convoyeur à bande de réception des insectes 19.

Le reste du mélange, qui est un mélange appauvri en insectes, tombe sur un deuxième convoyeur 2'. Le tri du mélange appauvri, à savoir la séparation des insectes encore présents dans le mélange appauvri, est réalisé comme cela est décrit en référence à la figure 2. De manière remarquable, la deuxième surface de réception 18' pour les insectes séparés par ce deuxième dispositif de tri est elle-même formée d'un deuxième convoyeur à bande de réception des insectes 19'.

Le reste du mélange appauvri, qui est quasiment ou totalement dépourvu d'insectes vivants, tombe sur un convoyeur à bande de collecte du reste 20, qui transporte ce reste jusqu'à, dans l'exemple représenté, un premier bac de récupération 11.

Le premier convoyeur à bande de réception des insectes 19 et le deuxième convoyeur à bande de réception des insectes 19' transportent les insectes afin de les collecter sur un convoyeur à bande de collecte des insectes 21. A l'extrémité de ce convoyeur à bande de collecte des insectes 21, les insectes tombent, dans l'exemple représenté, dans un deuxième bac de récupération 12.

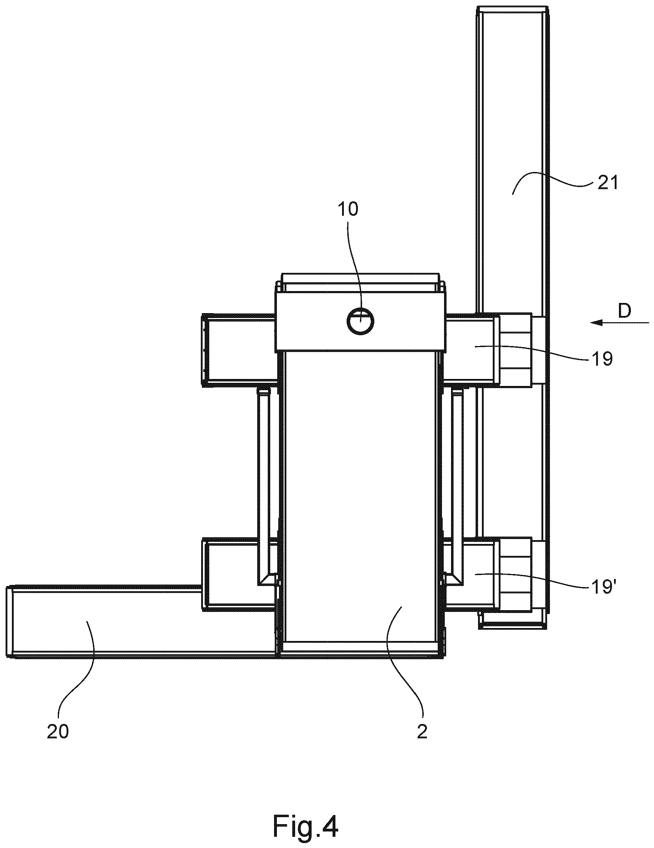

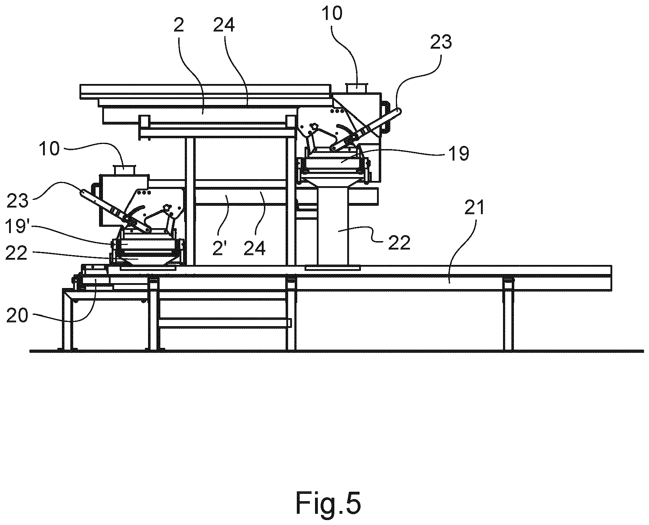

La figure 4 représente, selon un plan simplifié en vue de dessus, une machine correspondant à la machine de la figure 3. La figure 5 représente la machine de la figure 4, selon une vue de côté, selon la direction D mentionnée à la figure 4.

Les figures 4 et 5 permettent de visualiser un exemple concret de configuration d'une machine comportant deux dispositifs de tri successifs.

Ainsi, le convoyeur 2 et le deuxième convoyeur 2' sont superposés, à distance l'un de l'autre, et présentent un léger décalage longitudinal l'un par rapport à l'autre pour que le reste du mélange (c'est-à-dire le mélange appauvri en insectes) tombant par gravité du convoyeur 2 tombe sur le deuxième convoyeur 2'. Le convoyeur 2 et le deuxième convoyeur 2' sont configurés de sorte que la bande du convoyeur 2 et la bande du deuxième convoyeur 2' à bande sont entraînées en des directions opposées. Cela offre une meilleure compacité à la machine.

Le convoyeur 2 à bande et le deuxième convoyeur 2' à bande peuvent avoir, à titre d'exemple, une largeur de l'ordre de 30 cm à 1,5 m, par exemple de l'ordre de 1m. Le convoyeur 2 à bande et le deuxième convoyeur 2' à bande peuvent avoir, à titre d'exemple, une longueur totale comprise entre 1 m et 10 m, par exemple de l'ordre de 3 m. Ces dimensions sont également adaptées au mode de réalisation représenté à la figure 1.

Le premier convoyeur à bande de réception des insectes 19 et le deuxième convoyeur à bande de réception des insectes 19' s'étendent sensiblement orthogonalement auxdits convoyeur 2 et le deuxième convoyeur 2', dans des plans de hauteurs différentes, adaptées à la hauteur du convoyeur dont ils récupèrent les insectes. Au bout du premier convoyeur à bande de réception des insectes 19 et du deuxième convoyeur à bande de réception des insectes 19' est ménagée une colonne de descente 22 des insectes permettant de guider les insectes lors de leur chute sur le convoyeur à bande de collecte des insectes 21.

Dans l'exemple de machine de tri ici représenté, chacun des premier convoyeur 2 et deuxième convoyeur 2' comporte un levier de réglage 23 permettant de modifier l'inclinaison de la partie extrême du convoyeur à bande.

La machine représentée est équipée de hottes aspirantes, reliées aux embouchures de hottes 10. Pour contenir les poussières dans la machine, elle peut être équipée, comme dans l'exemple représenté, de carters 24 autour de chaque (ou de certains) convoyeur à bande. Les carters 24 sont avantageusement pourvus de moyens de démontage ou d'ouverture rapide, afin de faciliter le remplacement de la bande du convoyeur et de raccourcir la durée de cette opération.

Alternativement ou en complément, un carter général peut être réalisé autour de l'ensemble de la machine de tri.

De tels carters sont importants lorsque le ou les convoyeurs à bande sont équipés d'un dispositif de nettoyage par lame d'air comprimé. Un tel dispositif de nettoyage permet de nettoyer la bande d'un convoyeur en y projetant un flux d'air à haute pression, sur une surface très réduite et transversale à la bande. L'emploi de ce dispositif de nettoyage se révèle particulièrement avantageux dans un dispositif ou une machine conforme à l'invention, car il permet un nettoyage de la bande, et notamment un décolmatage de la face extérieure de la bande permettant l'accroche des insectes, sans abîmer la bande et même sans altérer l'état de surface de la bande, qui est fondamental pour assurer les propriétés d'accroche et donc de tri souhaitées.

Un dispositif de nettoyage par lame d'air comprimé 25 est représenté de manière schématique à la figure 1, pour en illustrer un positionnement préférentiel dans le dispositif. Le dispositif de nettoyage par lame d'air comprimé 25 est ainsi positionné sur la face inférieure du convoyeur, en aval du dispositif de séparation 13.

L'invention ainsi développée permet la réalisation de diverses opérations de tri dans un élevage d'insectes rampants à grande échelle. Elle permet une séparation efficace des insectes rampants (adultes ou larves dotées de pattes) présents dans un mélange du reste du mélange.

Comparativement aux machines de l'art antérieur fondées sur le même principe de tri selon lequel les insectes tendent à s'accrocher à une surface en mouvement sur laquelle ils évoluent, la présente invention améliore significativement l'efficacité du tri.

L'invention est ainsi particulièrement bien adaptée à la récupération des insectes adultes dans un mélange comportant des insectes adultes et des larves d'insectes, ainsi qu'à la récupération des insectes adultes vivants dans un mélange comportant des insectes adultes vivants et des insectes morts (qu'il s'agisse du même procédé de tri ou des procédés de tri distincts). Bien qu'applicable à de nombreuses espèces, elle trouve une application préférentielle dans un élevage de ténébrions meuniers.

## Revendications

1. Dispositif de tri d'insectes rampants ou essentiellement rampants, comportant une structure de support et :
- un convoyeur (2) à bande présentant une surface supérieure (5) et une surface inférieure (6) formée par la bande (3) du convoyeur (2), la bande (3) du convoyeur (2) comportant une face extérieure présentant une structure adaptée à l'accroche des pattes des insectes
- un dispositif d'entraînement configuré pour entraîner la bande (3) du convoyeur (2), de sorte que lors du fonctionnement du convoyeur (2) la bande (3) défile sur la surface supérieure d'une première extrémité (7) vers une deuxième extrémité (8) du convoyeur où la bande (3) est retournée pour passer sur la surface inférieure (6) du convoyeur (2) sur laquelle elle défile de la deuxième extrémité (8) vers la première extrémité (7) où la bande (3) est retournée pour passer de nouveau sur la surface supérieure (5), le dispositif permettant le dépôt d'un mélange comportant des insectes sur la surface supérieure (5) du convoyeur (2) à bande, de sorte que lors du retournement de la bande (3) au niveau de la deuxième extrémité (8) tout ou partie des insectes restent accrochés à la bande (3) tandis que le reste du mélange tombe par gravité,
- un dispositif de séparation des insectes permettant de détacher les insectes accrochés sur la surface inférieure (6) du convoyeur (2),
**caractérisé en ce que**, la surface supérieure (5) du convoyeur (2) étant sensiblement plate et horizontale sur une majorité de sa longueur entre la première extrémité (7) et la deuxième extrémité (8), ladite surface supérieure (5) du convoyeur (2) comporte une portion extrême (14), adjacente à la deuxième extrémité (8), présentant une inclinaison (α) vers le bas, comprise entre 20° et 90° vis-à-vis du reste de la surface supérieure (5) du convoyeur (2).

2. Dispositif selon la revendication 1, dans laquelle l'inclinaison (α) est comprise entre 30° et 70°, de préférence entre 45° et 60°.

3. Dispositif selon la revendication 1 ou la revendication 2, dans lequel la portion extrême (14) du convoyeur (2) présentant une inclinaison (α) a une longueur comprise entre vingt centimètres et un mètre, mesurée le long de sa surface supérieure.

4. Dispositif selon l'une des revendications précédentes, comportant un moyen de réglage de l'inclinaison (α).

5. Dispositif selon l'une quelconque des revendications précédentes, dans lequel la face extérieure de la bande (3) est formée d'un matériau tissé.

6. Dispositif selon la revendication 5, dans lequel le matériau tissé présente des mailles régulières sensiblement carrées ayant une ouverture de 110 microns à 1000 microns de côté.

7. Dispositif selon l'une des revendications précédentes, dans lequel le dispositif d'entraînement est configuré pour entraîner la bande (3) du convoyeur (2) à une vitesse de défilement comprise entre 10m/s et 30m/s.

8. Dispositif selon l'une quelconque des revendications précédentes, dans lequel le dispositif de séparation (13) est une lame ou une brosse s'étendant transversalement vis-à-vis de la bande (3), à proximité immédiate de sa surface.

9. Dispositif selon l'une quelconque des revendications précédentes comportant un système de nettoyage de la bande (3) générant une lame d'air comprimé venant impacter la bande (3) du convoyeur (2) au niveau de sa surface inférieure (6), entre le dispositif de séparation et la première extrémité (7) du convoyeur (2).

10. Dispositif selon l'une quelconque des revendications précédentes, comportant en outre une hotte aspirante adaptée à aspirer de l'air présent au-dessus de la surface supérieure (5) du convoyeur (2).

11. Dispositif selon l'une des revendications précédentes, comportant un carter enveloppant le convoyeur (2) de sorte à limiter la propagation de poussières depuis le convoyeur (2) vers l'extérieur du carter.

12. Machine de tri d'insectes comportant un premier dispositif de tri selon l'une des revendications précédentes et un deuxième dispositif selon l'une des revendications précédentes, dans laquelle ledit reste du mélange tombant par gravité du convoyeur (2) du premier dispositif de tri tombe directement sur, ou est amené sur, la surface supérieure (5) du convoyeur (2) du deuxième dispositif.

13. Utilisation d'un dispositif selon l'une des revendications 1 à 11 ou d'une machine selon la revendication 12 pour la récupération des insectes adultes dans un mélange comportant des insectes adultes et des larves d'insectes.

14. Utilisation d'un dispositif selon l'une des revendications 1 à 11 ou d'une machine selon la revendication 12 pour la récupération des insectes vivants dans un mélange comportant des insectes vivants et des insectes morts.

15. Utilisation selon la revendication 13 ou à la revendication 14, dans laquelle les insectes sont des ténébrions meuniers.

## Patentansprüche

1. Vorrichtung zum Sortieren von kriechenden oder überwiegend kriechenden Insekten, umfassend eine Stützstruktur und:
- einen Bandförderer (2), der eine obere Oberfläche (5) und eine untere Oberfläche (6), die durch das Band (3) des Förderers (2) gebildet werden, aufweist, wobei das Band (3) des Förderers (2) eine Außenfläche umfasst, die eine Struktur aufweist, die für das Aufhängen der Beine der Insekten ausgebildet ist,
- eine Antriebsvorrichtung, die dazu konfiguriert ist, das Band (3) des Förderers (2) anzutreiben, so dass bei Betrieb des Förderers (2) das Band (3) auf der oberen Oberfläche von einem ersten Ende (7) in Richtung eines zweiten Endes (8) des Förderers läuft, wo das Band (3) zurückgeführt wird, um auf die untere Oberfläche (6) des Förderers (2) zu gelangen, auf der es vom zweiten Ende (8) in Richtung des ersten Endes (7) läuft, wo das Band (3) zurückgeführt wird, um wieder auf die obere Oberfläche (5) zu gelangen, wobei die Vorrichtung das Ablegen einer Mischung aus Insekten auf der oberen Oberfläche (5) des Bandförderers (2) ermöglicht, so dass beim Zurückführen des Bands (3) auf Höhe des zweiten Endes (8) alle oder ein Teil der Insekten am Band (3) hängen bleiben, während der Rest der Mischung durch die Schwerkraft nach unten fällt,
- eine Vorrichtung zum Trennen der Insekten, die es ermöglicht, die an der unteren Oberfläche (6) des Förderers (2) hängenden Insekten abzulösen,
**dadurch gekennzeichnet, dass** die obere Oberfläche (5) des Förderers (2) über einen Großteil ihrer Länge zwischen dem ersten Ende (7) und dem zweiten Ende (8) im Wesentlichen flach und horizontal ist, wobei die obere Oberfläche (5) des Förderers (2) einen Endabschnitt (14) neben dem zweiten Ende (8) umfasst, der eine abwärts gerichtete Neigung (α) aufweist, die gegenüber dem Rest der oberen Oberfläche (5) des Förderers (2) zwischen 20° und 90° beträgt.

2. Vorrichtung nach Anspruch 1, wobei die Neigung (α) zwischen 30° und 70°, vorzugsweise zwischen 45° und 60°, beträgt.

3. Vorrichtung nach Anspruch 1 oder Anspruch 2, wobei der Endabschnitt (14) des Förderers (2) mit einer Neigung (α) eine Länge von zwischen zwanzig Zentimetern und einem Meter aufweist, gemessen entlang seiner oberen Oberfläche.

4. Vorrichtung nach einem der vorhergehenden Ansprüche, umfassend ein Mittel zum Einstellen der Neigung (α).

5. Vorrichtung nach einem der vorhergehenden Ansprüche, wobei die Außenseite des Bands (3) aus einem gewebten Material gebildet ist.

6. Vorrichtung nach Anspruch 5, wobei das gewebte Material regelmäßige, im Wesentlichen quadratische Maschen auf, die eine Öffnung von 110 Mikrometer bis 1000 Mikrometer Seitenlänge haben.

7. Vorrichtung nach einem der vorhergehenden Ansprüche, wobei die Antriebsvorrichtung dazu konfiguriert ist, das Band (3) des Förderers (2) mit einer Laufgeschwindigkeit zwischen 10 m/s und 30 m/s anzutreiben.

8. Vorrichtung nach einem der vorhergehenden Ansprüche, wobei die Trennvorrichtung (13) eine Klinge oder eine Bürste ist, die sich quer zum Band (3) in unmittelbarer Nähe seiner Oberfläche erstreckt.

9. Vorrichtung nach einem der vorhergehenden Ansprüche, umfassend ein System zum Reinigen des Bands (3), das ein Messer aus Druckluft erzeugt, das auf das Band (3) des Förderers (2) auf Höhe seiner unteren Oberfläche (6) zwischen der Trennvorrichtung und dem ersten Ende (7) des Förderers (2) trifft.

10. Vorrichtung nach einem der vorhergehenden Ansprüche, ferner umfassend eine Abzugshaube, die dazu ausgebildet ist, die Luft, die über der obere Oberfläche (5) des Förderers (2) vorhanden ist, anzusaugen.

11. Vorrichtung nach einem der vorhergehenden Ansprüche, umfassend ein Gehäuse, das den Förderer (2) umhüllt, um die Ausbreitung von Staub vom Förderer (2) auf die Außenseite des Gehäuses zu begrenzen.

12. Maschine zum Sortieren von Insekten, umfassend eine erste Sortiervorrichtung nach einem der vorhergehenden Ansprüche und eine zweite Vorrichtung nach einem der vorhergehenden Ansprüche, wobei der Rest der Mischung durch die Schwerkraft vom Förderer (2) der ersten Sortiervorrichtung direkt auf die obere Oberfläche (5) des Förderers (2) der zweiten Vorrichtung fällt oder dorthin geleitet wird.

13. Verwendung einer Vorrichtung nach einem der Ansprüche 1 bis 11 oder einer Maschine nach Anspruch 12 zur Gewinnung von erwachsenen Insekten aus einer Mischung, die erwachsene Insekten und Insektenlarven umfasst.

14. Verwendung einer Vorrichtung nach einem der Ansprüche 1 bis 11 oder einer Maschine nach Anspruch 12 zur Gewinnung von lebenden Insekten aus einer Mischung, die lebende Insekten und tote Insekten umfasst.

15. Verwendung nach Anspruch 13 oder Anspruch 14, wobei die Insekten Mehlkäfer sind.

## Claims

1. Device for sorting crawling insects, or insects that are primarily crawling insects, comprising a support structure and:
- a belt conveyor (2) having an upper surface (5) and a lower surface (6) formed by the belt (3) of the conveyor (2), the belt (3) of the conveyor (2) comprising an outside face having a structure configured for the attachment of the insects' legs
- a driving device configured to drive the belt (3) of the conveyor (2), such that on operation of the conveyor (2) the belt (3) runs on the upper surface of a first end (7) towards a second end (8) of the conveyor where the belt (3) is turned over to pass on the lower surface (6) of the conveyor (2) on which it runs from the second end (8) towards the first end (7) where the belt (3) is turned over to pass again on the upper surface. (5), the device enabling the deposit of a mixture comprising insects on the upper surface (5) of the belt conveyor (2), such that on turning over of the belt (3) at the location of the second end (8), all or some of the insects remain attached to the belt (3) while the rest of the mixture falls by gravity,
- a device for separating the insects making it possible to detach the insects attached to the lower surface (6) of the conveyor (2),
**characterized in that**, as the upper surface (5) of the conveyor (2) being substantially flat and horizontal over a majority of its length between the first end (7) and the second end (8), said upper surface (5) of the conveyor (2) comprises an end portion (14), adjacent the second end (8), having a downward inclination (α), comprised between 20° and 90° relative to the rest of the upper surface (5) of the conveyor (2).

2. Device according to claim 1, wherein the inclination (α) is comprised between 30° and 70°, preferably between 45° and 60°.

3. Device according to claim 1 or claim 2, wherein the end portion (14) of the conveyor (2) having an inclination (α) has a length comprised between twenty centimeters and one meter, measured along its upper surface.

4. Device according to one of the preceding claims, comprising a means for adjusting the inclination (α).

5. Device according to any one of the preceding claims, wherein the outside face of the belt (3) is formed from a woven material.

6. Device according to claim 5, wherein the woven material has regular substantially square mesh elements having an opening of which the sides measure 110 microns to 1000 microns.

7. Device according to one of the preceding claims, wherein the driving device is configured to drive the belt (3) of the conveyor (2) at a running speed comprised between 10m/s and 30m/s.

8. Device according to any one of the preceding claims, wherein the separating device (13) is a blade or a brush extending transversely in relation to the belt (3), in immediate proximity to its surface.

9. Device according to any one of the preceding claims, comprising a system for cleaning the belt (3) generating a blade of compressed air that comes to impinge upon the belt (3) of the conveyor (2) at the location of its lower surface (6), between the separating device and the first end (7) of the conveyor (2).

10. A device according to any one of the preceding claims, further comprising an extractor hood configured to suck away the air present above the upper surface (5) of the conveyor (2).

11. Device according to one of the preceding claims comprising a casing enveloping the conveyor (2) so as to limit the propagation of dust from the conveyor (2) to outside the casing.

12. Machine for sorting insects comprising a first sorting device according to any one of the preceding claims and a second device according to any one of the preceding claims, in which said rest of the mixture falling by gravity from the conveyor (2) of the first sorting device falls directly onto, or is brought onto, the upper surface (5) of the conveyor (2) of the second device.

13. Use of a device according to one of claims 1 to 11 or of a machine according to claim 12, for the recovery of the adult insects from a mixture comprising adult insects and insect larvae.

14. Use of a device according to one of claims 1 to 11 or of a machine according to claim 12, for the recovery of the live insects from a mixture comprising live insects and dead insects.

15. Use according to claim 13 or claim 14, wherein the insects are yellow mealworm.
